# EUROPEAN PATENT SPECIFICATION

(11) **EP 2 482 845 B1**
(45) Date of publication and mention of the grant of the patent: **25.01.2017**
(21) Application number: 10765603.5
(22) Date of filing: 28.09.2010
(51) Int. Cl.: A61K 39/145

(54) **NOVEL VACCINE COMPOSITION AGAINST INFLUENZA**
NEUE IMPFSTOFFZUSAMMENSETZUNG GEGEN INFLUENZA
NOUVELLE COMPOSITION DE VACCIN CONTRE LA GRIPPE

(30) Priority: 30.09.2009 US 570609
(43) Date of publication of application: 08.08.2012
(73) Proprietor: Glaxosmithkline Biologicals, Niederlassung Der Smithkline Beecham Pharma GmbH & Co. KG, 01069 Dresden (DE)
(72) Inventor: EICHHORN, Uwe, 01069 Dresden (DE); SAENGER, Roland Herbert, 80339 München (DE)
(74) Representative: Van Den Hazel, Hendrik Bart
(86) International application number: PCT/EP2010/064352
(87) International publication number: WO 2011/039180

(56) References cited:
- WO-A1-2006/100110
- WO-A1-2008/128939
- WO-A2-2010/092479
- US-A1- 2008 181 914
- BALL L K ET AL: "AN ASSESSMENT OF THIMEROSAL USE IN CHILDHOOD VACCINES", PEDIATRICS, AMERICAN ACADEMY OF PEDIATRICS, EVANSTON, IL, US, vol. 107, no. 5, 1 May 2001 (2001-05-01), pages 1147-1154, XP009001918, ISSN: 0031-4005, DOI: DOI:10.1542/PEDS.107.5.1147
- CARMONA ALFONSO ET AL: "Immunogenicity and safety of AS03-adjuvanted 2009 influenza A H1N1 vaccine in children 6-35 months.", VACCINE 16 AUG 2010 LNKD- PUBMED:20600478, vol. 28, no. 36, 16 August 2010 (2010-08-16), pages 5837-5844, XP002628057, ISSN: 1873-2518
- CAMILLONI B ET AL: "Cross-reactive antibodies in middle-aged and elderly volunteers after MF59-adjuvanted subunit trivalent influenza vaccine against B viruses of the B/Victoria or B/Yamagata lineages", VACCINE, ELSEVIER LTD, GB, vol. 27, no. 31, 24 June 2009 (2009-06-24) , pages 4099-4103, XP026158064, ISSN: 0264-410X, DOI: 10.1016/J.VACCINE.2009.04.078 [retrieved on 2009-05-14]

## Description

This application is a continuation-in-part of co-pending US Application No. 11/874,647, filed 18 October 2007, which is a continuation of Application No. 10/480,952, filed 22 June 2004, now US Patent No. 7,316,813, which is a 371 of International Application No. PCT/EP02/05833, filed 29 May 2002. This application also claims benefit of the earlier filing dates of UK Patent Applications No. 0113083.0, filed 30 May 2001 and 0204116.8, filed 21 February 2002.

This invention relates to novel influenza virus antigen preparations, methods for preparing them and their use in prophylaxis or therapy of human subjects. In particular the invention relates to inactivated influenza vaccines which are disrupted rather than whole virus vaccines and which are stable in the absence of organomercurial preservatives. Moreover, the vaccines contain haemagglutinin which is stable according to standard tests. This invention further relates to the use of mercurial preservative-free influenza immunogenic compositions in immunization of children. A mercurial preservative-free influenza immunogenic composition is eliciting higher immune response in the paediatric population compared to a composition containing organomercurial preservatives. The vaccines can be administered by any route suitable for such vaccines, such as intramuscularly, subcutaneously, intradermally or mucosally e.g. intranasally.

Influenza virus is one of the most ubiquitous viruses present in the world, affecting both humans and livestock. The economic impact of influenza is significant. Influenza viruses circulate most winters in temperate regions and throughout the year in tropical regions. Influenza A and B possess the surface glycoproteins haemagglutinin (HA) and neuraminidase (NA), which evolve from year to year in a process of point mutations, known as antigenic drift. This continuous alteration of the influenza viruses allows them to evade the host immune system and is the reason why seasonal influenza vaccination must be reiterated every year.

The influenza virus is an RNA enveloped virus with a particle size of about 125 nm in diameter. It consists basically of an internal nucleocapsid or core of ribonucleic acid (RNA) associated with nucleoprotein, surrounded by a viral envelope with a lipid bilayer structure and external glycoproteins. The inner layer of the viral envelope is composed predominantly of matrix proteins and the outer layer mostly of host-derived lipid material. The surface glycoproteins neuraminidase (NA) and haemagglutinin (HA) appear as spikes, 10 to 12 nm long, at the surface of the particles. It is these surface proteins, particularly the haemagglutinin, that determine the antigenic specificity of the influenza subtypes.

Currently available influenza vaccines are either inactivated or live attenuated influenza vaccine. Inactivated flu vaccines are composed of three possible forms of antigen preparation: inactivated whole virus, sub-virions where purified virus particles are disrupted with detergents or other reagents to solubilise the lipid envelope (so-called "split" vaccine) or purified HA and NA (subunit vaccine). These inactivated vaccines are given intramuscularly (i.m.) or intranasaly (i.n.). There is no commercially available live attenuated vaccine.

Influenza vaccines, of all kinds, are usually trivalent vaccines. They generally contain antigens derived from two influenza A virus strains and one influenza B strain. A standard 0.5 ml injectable dose in most cases contains 15 µg of haemagglutinin antigen component from each strain, as measured by single radial immunodiffusion (SRD) (J.M. Wood et al.: An improved single radial immunodiffusion technique for the assay of influenza haemagglutinin antigen: adaptation for potency determination of inactivated whole virus and subunit vaccines. J. Biol. Stand. 5 (1977) 237-247; J. M. Wood et al., International collaborative study of single radial diffusion and immunoelectrophoresis techniques for the assay of haemagglutinin antigen of influenza virus. J. Biol. Stand. 9 (1981) 317-330).

The influenza virus strains to be incorporated into influenza vaccine each season are determined by the World Health Organisation in collaboration with national health authorities and vaccine manufacturers.

Typical influenza epidemics cause increases in incidence of pneumonia and lower respiratory disease as witnessed by increased rates of hospitalisation or mortality. The elderly or those with underlying chronic diseases are most likely to experience such complications, but young infants also may suffer severe disease. There is growing evidence that influenza has a significant impact on children. The highest attack rates of influenza occur in children and the rates of influenza-related morbidity are highest in this age group. Children are also considered to be the main transmitters of influenza, as they suffer the highest attack rates from the virus, have the highest nasopharyngeal titres of the virus and show a longer duration of viral shedding. Vaccinating children against influenza would therefore prevent the serious morbidity associated with the disease and could also have an impact on the "herd effect" and decrease the impact of influenza on the community. Apart from the direct effects of vaccination on the health of the children themselves, their household contacts and the wider community, there is also a strong economic factor involved. Indeed, the morbidity associated with influenza is responsible for significant rates of absenteeism and loss of productivity. These groups in particular therefore need to be protected.

Current efforts to control the morbidity and mortality associated with yearly epidemics of influenza are based on the use of intramuscularly administered inactivated influenza vaccines. The efficacy of such vaccines in preventing respiratory disease and influenza complications ranges from 75% in healthy adults to less than 50% in the elderly.

Standards are applied internationally to measure the efficacy of influenza vaccines. The European Union official criteria for an effective vaccine against influenza are set out in the table below. Theoretically, to meet the European Union requirements, an influenza vaccine has to meet only one of the criteria in the table, for all strains of influenza included in the vaccine. However in practice, at least two or all three of the criteria will need to be met for all strains, particularly for a new vaccine such as a new vaccine for delivery via a different route. Under some circumstances two criteria may be sufficient. For example, it may be acceptable for two of the three criteria to be met by all strains while the third criterion is met by some but not all strains (e.g. two out of three strains). The requirements are different for adult populations (18-60 years) and elderly populations (>60 years).

| | 18 - 60 years | > 60 years |
|---|---|---|
| Seroconversion rate* | >40% | >30% |
| Conversion factors* | >2.5 | >2.0 |
| Protection rate*** | >70% | >60% |

| | | |
|---|---|---|
| * Seroconversion rate is defined as the percentage of vaccinees who have at least a 4-fold increase in serum haemagglutinin inhibition (HI) titres after vaccination, for each vaccine strain. ** Conversion factor is defined as the fold increase in serum HI geometric mean titres (GMTs) after vaccination, for each vaccine strain. *** Protection rate is defined as the percentage of vaccinees with a serum HI titre equal to or greater than 1:40 after vaccination (for each vaccine strain) and is normally accepted as indicating protection. | | |

FDA uses slightly different age cut-off points, but their criteria are based on the CHMP criteria. Appropriate endpoints similarly include: 1) the percent of subjects achieving an HI antibody titer ≥ 1:40, and 2) rates of seroconversion, defined as a four-fold rise in HI antibody titer post-vaccination. The geometric mean titer (GMT) should be included in the results, but the data should include not only the point estimate, but also the lower bound of the 95% confidence interval of the incidence rate of seroconversion, and the day 42 incidence rate of HI titers ≥ 1:40 must exceed the target value. These data and the 95% confidence intervals (CI) of the point estimates of these evaluations should therefore be provided. FDA draft guidance requires that both targets be met. This is summarised in Table 1B.

| | 18 - 64 years | > 64 years |
|---|---|---|
| Seroconversion rate * | >40% | >30% |
| Rate of HI titers ≥ 1:40 | >70% | >60% |

| | | |
|---|---|---|
| * The seroconversion rate is is defined as: a) for subjects with a baseline titer ≥ 1:10, a 4-fold or greater rise; or b) for subjects with a baseline titer < 1:10, a rise to ≥ 1:40. These criteria must be met at the lower bound of the 95% CI for the true value. | | |

For a novel flu vaccine to be commercially useful it will not only need to meet those standards, but also in practice it will need to be at least as efficacious as the currently available injectable vaccines. It will also need to be commercially viable in terms of the amount of antigen and the number of administrations required.

The current commercially available influenza vaccines are either split or subunit injectable vaccines. These vaccines are prepared by disrupting the virus particle, generally with an organic solvent or a detergent, and separating or purifying the viral proteins to varying extents. Split vaccines are prepared by fragmentation of whole influenza virus, either infectious or inactivated, with solubilizing concentrations of organic solvents or detergents and subsequent removal of the solubilizing agent and some or most of the viral lipid material. Split vaccines generally contain contaminating matrix protein and nucleoprotein and sometimes lipid, as well as the membrane envelope proteins. Split vaccines will usually contain most or all of the virus structural proteins although not necessarily in the same proportions as they occur in the whole virus. Examples of commercially available split vaccines are for example FLUARIX™, FLUSHIELD™, or FLUZONE™
Subunit vaccines on the other hand consist essentially of highly purified viral surface proteins, haemagglutinin optionally with neuraminidase, which are the surface proteins responsible for eliciting the desired virus neutralising antibodies upon vaccination. Subunit vaccines may have an additional advantage over whole virion vaccines as they are generally less reactogenic, particularly in young vaccinees. Sub-unit vaccines can be produced either recombinantly or purified from disrupted viral particles. Examples of commercially available sub-unit vaccines are for example AGRIPPAL™, or FLUVIRIN™. In a specific embodiment, sub-unit vaccines are prepared from at least one major envelope component such as from haemagglutinin (HA), neuraminidase (NA), or M2, suitably from HA. Suitably they comprise combinations of two antigens or more, such as a combination of at least two of the influenza structural proteins HA, NA, Matrix 1 (M1) and M2, suitably a combination of both HA and NA, optionally comprising M1. Suitably, the influenza components are produced by recombinant DNA technology, i.e. results from, or is expressed from, a nucleic acid resulting from recombinant DNA manipulations, including live recombinant vector (vaccinia) or recombinant subunit protein (baculovirus/insect cells, mammalian cells, avian cells, yeast, plants or bacteria). Suitable insect cells are *Spodoptera frugiperda* (Sf9) insect cells or High Five (Hi5) insect cells developed from *Trichoplusia ni* (Invitrogen) and suitable baculovirus are Autographa californica nuclear polyhedrosis virus (AcNPV) (Baculogold, Becton Dickinson, PharMingen) or the so-called Bacmid system.
In one embodiment, the influenza virus preparation is in the form of a virosome. Virosomes are spherical, unilamellar vesicles which retain the functional viral envelope glycoproteins HA and NA in authentic conformation, intercalated in the virosomes' phospholipids bilayer membrane. Examples of commercially available virosomal vaccines are for example INFLEXAL V™, or INVAVAC™.
In another embodiment, the sub-unit influenza components are expressed in the form of virus-like-particles (VLP) or capsomers, suitably plant-made or insect cells-made VLPs. VLPs present the antigens in their native form. The VLP sub-unit technology may be based entirely on influenza proteins, or may rely on other virus such as the murine leukaemia virus (MLV) and may therefore comprise a non-influenza antigen such as MLV gag protein. A suitable VLP comprises at least one, suitably at least two influenza proteins, optionally with other influenza or non-influenza proteins, such as M1 and HA, HA and NA, HA, NA and M1 or HA, NA and MLV gag. It may be produced either in plant cells or insect cells. VLPs can also carry antigens from more than one influenza strain, such as VLPs made from two seasonal strains (e.g. H1N1 and H3N2) or from one seasonal and one pandemic strain (e.g. H3N2 and H5N1) for example.

Many vaccines which are currently available require a preservative to prevent deterioration. A frequently used preservative is thimerosal which is a mercury-containing compound. Some public concerns have been expressed about the effects of mercury containing compounds. There is no surveillance system in place to detect the effects of low to moderate doses of organomercurials on the developing nervous system, and special studies of children who have received high doses of organomercurials will take several years to complete. Certain commentators have stressed that the potential hazards of thimerosal-containing vaccines should not be overstated (Offit; P.A. JAMA Vol.283;No:16). Nevertheless, it would be advantageous to find alternative methods for the preparation of vaccines to replace the use of thiomerosal in the manufacturing process. There is thus a need to develop vaccines which are thimerosal-free, in particular vaccines like influenza vaccines which are recommended, at least for certain population groups, on an annual basis.

It has been standard practice to date to employ a preservative for commercial inactivated influenza vaccines, during the production/purification process and/or in the final vaccine. The preservative is required to prevent microorganisms from growing through the various stages of purification. For egg-derived influenza vaccines, thiomersal is typically added to the raw allantoic fluid and may also be added a second time during the processing of the virus. Thus there will be residual thiomersal present at the end of the process, and this may additionally be adjusted to a desirable preservative concentration in the final vaccine, for example to a concentration of around 100 µg/ml.

A side-effect of the use of thiomersal as a preservative in flu vaccines is a stabilisation effect. The thiomersal in commercial flu vaccines acts to stabilise the HA component of the vaccine, in particular but not exclusively HA of B strain influenza. Certain A strain haemagglutinins e.g. H3 may also require stabilisation. Therefore, although it may be desirable to consider removing thiomersal from influenza vaccines, or at least reducing the concentration of the thiomersal in the final vaccine, there is a problem to overcome in that, without thiomersal, the HA will not be sufficiently stable.

WO2006/100110 and WO2008/128939 disclose clinical studies in adult or elderly populations testing adjuvanted mercurial-preservative-free influenza vaccines comprising haemagglutinin and α-tocopherol succinate in an amount sufficient to stabilize said HA.

It has been discovered in the present invention that it is possible to stabilise HA in inactivated influenza preparations using alternative reagents that do not contain organomercurials. The HA remains stabilised such that it is detectable over time by quantitative standard methods, in particular SRD, to an greater extent than a non-stabilised antigen preparation produced by the same method but without stabilising excipient. The SRD method is performed as described hereinabove. Importantly, the HA remains stabilised for up to 12 months which is the standard required of a final flu vaccine.
It has also been surprisingly found that a mercurial preservative-free influenza vaccine elicits significantly improved immune responses in children. Compared to a thiomersal-containing influenza vaccine the thiomersal-free vaccine induced in children higher antibody titres (GMT), for all strains tested, and in particular for the H1N1 influenza strain where the effect was especially marked. Furthermore the three CHMP criteria for the immunogenicity assessment of influenza vaccines in adults were only met for both children aged 6 to 35 months and those aged 36 months to <6 years in the TF group. In the control group, only the SCR and SCF criteria were met for both age groups.

In a first aspect the present invention provides a mercurial preservative-free immunogenic composition for use in prophylaxis of influenza infection or disease in a child of between 6 months and 36 months of age, wherein said composition comprises an aqueous inactivated influenza virus preparation comprising haemagglutinin (HA) and α-tocopherol succinate in an amount sufficient to stabilize said HA, wherein the preparation is a split or sub-unit virus antigen preparation and is a trivalent influenza virus preparation comprising 2 A strains and 1 B strain HA or a tetravalent influenza virus preparation comprising 2 A strains and 2 B strains HA and wherein the composition does not comprise an adjuvant.

Low levels of thiomersal are those levels at which the stability of HA derived from influenza B is reduced, such that a stabilising excipient is required for stabilised HA Low levels of thiomersal are generally 5 µg/ml or less.

Generally, stabilised HA refers to HA which is detectable over time by quantitative standard methods, in particular SRD, to an greater extent than a non-stabilised antigen preparation produced by the same method but without any stabilising excipient. Stabilisation of HA preferably maintains the activity of HA substantially constant over a one year period. Preferably, stabilisation allows the vaccine comprising HA to provide acceptable protection after a 6 month storage period, more preferably a one year period.

Suitably, stabilisation is carried out by a stabilising excipient, preferably a micelle modifying excipient. A micelle modifying excipient is generally an excipient that may be incorporated into a micelle formed by detergents used in, or suitable for, solubilising the membrane protein HA, such as the detergents Tween 80, Triton X100 and deoxycholate, individually or in combination.

Without wishing to be constrained by theory, it is believed that the excipients work to stabilise HA by interaction with the lipids, detergents and/or proteins in the final preparation. Mixed micelles of excipient with protein and lipid may be formed, such as micelles of Tween and deoxycholate with residual lipids and/or Triton X-100. It is thought that surface proteins are kept solubilised by those complex micelles. Preferably, protein aggregation is limited by charge repulsion of micelles containing suitable excipients, such as micelles containing negatively charged detergents.

Suitable micelle modifying excipients include: positively, negatively or zwitterionic charged amphiphilic molecules such as alkyl sulfates, or alkyl-aryl- sulfates; non-ionic amphiphilic molecules such as alkyl polyglycosides or derivatives thereof, such as Plantacare® (available from Henkel KGaA), or alkyl alcohol poly alkylene ethers or derivatives thereof such as Laureth-9.

Alpha-tocopherol succinate is particularly preferred. The α-tocopherol succinate is present in an amount sufficient to stabilise the haemagglutinin.

Other suitable excipients may be identified by methods standard in the art, and tested for example using the SRD method for stability analysis as described herein.

A vaccine dose of 0.5 ml is suitably used. For the paediatric population, a vaccine dose of less than 0.5 ml is used. A suitable dose is between 0.2-0.45 ml, or between 0.2-0.3ml, typically about 0.25ml. Slight adaptation of the dose volume will be made routinely depending on the HA concentration in the original bulk sample, or depending on the delivery route with smaller doses being given by the intranasal or intradermal route, or depending on the target population (for example infants between 0-35 months may receive a 0.25 ml vaccine dose, when children from the age of 3 may receive a higher vaccine dose).

In another embodiment, the target population to vaccinate is children between 6 months and 36 months of age, especially children 6-23 months of age who experience a relatively high influenza-related hospitalization rate.

The vaccine may be administered by any suitable delivery route, such as intradermal, mucosal e.g. intranasal, oral, intramuscular or subcutaneous. Other delivery routes are well known in the art.

Intradermal delivery is preferred. Any suitable device may be used for intradermal delivery, for example short needle devices such as those described in US 4,886,499, US5,190,521, US 5,328,483, US 5,527,288, US 4,270,537, US 5,015,235, US 5,141,496, US 5,417,662. Intradermal vaccines may also be administered by devices which limit the effective penetration length of a needle into the skin, such as those described in WO99/34850 and EP1092444, incorporated herein by reference, and functional equivalents thereof. Also suitable are jet injection devices which deliver liquid vaccines to the dermis via a liquid jet injector or via a needle which pierces the stratum corneum and produces a jet which reaches the dermis. Jet injection devices are described for example in US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556US 4,790,824, US 4,941,880, US 4,940,460, WO 97/37705 and WO 97/13537. Also suitable are ballistic powder/particle delivery devices which use compressed gas to accelerate vaccine in powder form through the outer layers of the skin to the dermis. Additionally, conventional syringes may be used in the classical mantoux method of intradermal administration. However, the use of conventional syringes requires highly skilled operators and thus devices which are capable of accurate delivery without a highly skilled user are preferred.

The disclosure further provides a method for the prophylaxis of influenza infection or disease in a subject which method comprises administering to the subject intradermally an influenza vaccine according to the invention.

The invention also extends to intradermal devices in combination with the use of a vaccine according to the present invention, in particular with devices disclosed in WO99/34850 or EP1092444, for example.

The invention applies particularly but not exclusively to the stabilisation of B strain influenza haemagglutinin.

Preferably the stabilised HA of the present invention is stable for 6 months, more preferably 12 months.

Preferred concentrations for the α-tocopherol or derivative are between 1 µg/ml-10mg/ml, more preferably between 10µg/ml - 500 µg/ml.

The vaccine according to the invention generally contains both A and B strain virus antigens, typically in a trivalent composition of two A strains and one B strain. Quadrivalent vaccines are also considered, in particular vaccines comprising: (i) two A strains (e.g. H3N2 and H1N1) and two B strains of a different lineage (e.g. B/Yamagata and B/Victoria), (ii) three A strains (e.g. H3N2, two H1N1; or H3N2, H1N1, H5N1) and one B strain.

The HA component in the immunogenic composition may be selected from the group consisting of: H1, H2, H3, H5, H7, and H9.

In one embodiment, each paediatric dose of the immunogenic composition contains 15 µg HA per influenza strain, as measured by single radial immunodiffusion (SRD) (J.M. Wood et al.: J. Biol. Stand. 5 (1977) 237-247; J. M. Wood et al., J. Biol. Stand. 9 (1981) 317-330). In another embodiment, a low dose of haemagglutinin (HA) is used, defined as an amount of less than 15 µg of HA per dose, suitably less than 10 µg,. In a specific embodiment, the paediatric dose of the immunogenic composition comprises a dose of haemagglutinin (HA) per strain at a level of about 10 µg, for example between 5 and 15 µg, suitably between 6 and 14 µg, for example between 7 and 13 µg or between 8 and 12 µg or between 9 and 11 µg, or 10 µg. In a further embodiment, the human dose of the immunogenic composition comprises a dose of haemagglutinin (HA) per strain at a level of about 5 µg, for example between 1 and 9 µg, or between 2 and 8 µg or suitably between 3 and 7 µg or 4 and 6 µg, or 5 µg. Suitable amounts are 1.9 µg, 2.5 µg, 3.8 µg, 5.0 µg, 7.5 µg, or 10 µg HA or any suitable amount of HA lower than 15 µg which would have be determined such that the vaccine composition meets the efficacy criteria as defined herein. Advantageously an HA dose of 1 µg of HA or even less such as 0.5 µg of HA that would allow meeting the regulatory criteria may be used. A suitable amount of HA is for example any of 1, 2, 3, 4, 5, 6, 7, 8, 9, 10, 11, 12, 13, 14, µg (w/v) per influenza strain per human dose of the immunogenic composition. Said low amount of HA may be as low as practically feasible provided that it allows to formulate a vaccine which meets the international e.g. EU or FDA criteria for efficacy.

The non-live flu antigen preparation for use in the invention may be selected from the group consisting of split virus antigen preparations and subunit antigens (either recombinantly expressed or prepared from whole virus). The antigen preparation is either a split virus preparation, or a subunit antigen prepared from whole virus, particularly by a splitting process followed by purification of the surface antigen. Most preferred are split virus preparations. Other preferred preparations are subunit virus preparations.

Preferably the concentration of haemagglutinin antigen for the or each strain of the influenza virus preparation is 1-1000 µg per ml, more preferably 3-300 µg per ml and most preferably about 30 µg per ml, as measured by a SRD assay.

The vaccines according to the invention may further comprise at least one surfactant which may be in particular a non-ionic surfactant. Suitable non-ionic surfactant are selected from the group consisting of the octyl- or nonylphenoxy polyoxyethanols (for example the commercially available Triton ™ series), polyoxyethylene sorbitan esters (Tween™ series) and polyoxyethylene ethers or esters of general formula (I):

(I) HO(CH₂CH₂O)ₙ-A-R

wherein n is 1-50, A is a bond or -C(O)-, R is C₁₋₅₀ alkyl or phenyl C₁₋₅₀ alkyl; and combinations of two or more of these.

Preferred surfactants falling within formula (I) are molecules in which n is 4-24, more preferably 6-12, and most preferably 9; the R component is C₁₋₅₀, preferably C₄₋C₂₀ alkyl and most preferably C₁₂ alkyl.

Octylphenoxy polyoxyethanols and polyoxyethylene sorbitan esters are described in "Surfactant systems" Eds: Attwood and Florence (1983, Chapman and Hall). Octylphenoxy polyoxyethanols (the octoxynols), including t-octylphenoxypolyethoxyethanol (Triton X-100 ™) are also described in Merck Index Entry 6858 (Page 1162, 12^{th} Edition, Merck & Co. Inc., Whitehouse Station, N.J., USA; ISBN 0911910-12-3). The polyoxyethylene sorbitan esters, including polyoxyethylene sorbitan monooleate (Tween 80 ™) are described in Merck Index Entry 7742 (Page 1308, 12^{th} Edition, Merck & Co. Inc., Whitehouse Station, N.J., USA; ISBN 0911910-12-3). Both may be manufactured using methods described therein, or purchased from commercial sources such as Sigma Inc.

Particularly preferred non-ionic surfactants include Triton X-45, t-octylphenoxy polyethoxyethanol (Triton X-100), Triton X-102, Triton X-114, Triton X-165, Triton X-205, Triton X-305, Triton N-57, Triton N-101, Triton N-128, Breij 35, polyoxyethylene-9-lauryl ether (laureth 9) and polyoxyethylene-9-stearyl ether (steareth 9). Triton X-100 and laureth 9 are particularly preferred. Also particularly preferred is the polyoxyethylene sorbitan ester, polyoxyethylene sorbitan monooleate (Tween 80™).

Further suitable polyoxyethylene ethers of general formula (I) are selected from the following group: polyoxyethylene-8-stearyl ether, polyoxyethylene-4-lauryl ether, polyoxyethylene-35-lauryl ether, and polyoxyethylene-23-lauryl ether.

Alternative terms or names for polyoxyethylene lauryl ether are disclosed in the CAS registry. The CAS registry number of polyoxyethylene-9 lauryl ether is: 9002-92-0. Polyoxyethylene ethers such as polyoxyethylene lauryl ether are described in the Merck index (12^{th} ed: entry 7717, Merck & Co. Inc., Whitehouse Station, N.J., USA; ISBN 0911910-12-3). Laureth 9 is formed by reacting ethylene oxide with dodecyl alcohol, and has an average of nine ethylene oxide units.

Two or more non-ionic surfactants from the different groups of surfactants described may be present in the vaccine formulation described herein. In particular, a combination of a polyoxyethylene sorbitan ester such as polyoxyethylene sorbitan monooleate (Tween 80 ™) and an octoxynol such as t-octylphenoxypolyethoxyethanol (Triton) X-100™ is preferred. Another particularly preferred combination of non-ionic surfactants comprises laureth 9 plus a polyoxyethylene sorbitan ester or an octoxynol or both.

Non-ionic surfactants such as those discussed above have preferred concentrations in the final vaccine composition as follows: polyoxyethylene sorbitan esters such as Tween 80™: 0.01 to 1%, most preferably about 0.1% (w/v); octyl- or nonylphenoxy polyoxyethanols such as Triton X-100™ or other detergents in the Triton series: 0.001 to 0.1%, most preferably 0.005 to 0.02 % (w/v); polyoxyethylene ethers of general formula (I) such as laureth 9: 0.1 to 20 %, preferably 0.1 to 10 % and most preferably 0.1 to 1 % or about 0.5% (w/v).

For certain vaccine formulations, other vaccine components may be included in the formulation. As such the formulations of the present invention may also comprise a bile acid or a derivative thereof, in particular in the form of a salt. These include derivatives of cholic acid and salts thereof, in particular sodium salts of cholic acid or cholic acid derivatives. Examples of bile acids and derivatives thereof include cholic acid, deoxycholic acid, chenodeoxycholic acid, lithocholic acid, ursodeoxycholic acid, hyodeoxycholic acid and derivatives such as glyco-, tauro-, amidopropyl-1-propanesulfonic-, amidopropyl-2-hydroxy-1-propanesulfonic derivatives of the aforementioned bile acids, or N,N-bis (3Dgluconoamidopropyl) deoxycholamide. A particularly preferred example is sodium deoxycholate (NaDOC) which may be present in the final vaccine dose.

Also provided by the disclosure are pharmaceutical kits comprising a vaccine administration device filled with a vaccine for use according to the invention. Such administration devices include but are not limited to needle devices, liquid jet devices, powder devices, and spray devices (for intranasal use).

The influenza virus antigen preparations according to the invention may be derived from the conventional embryonated egg method, or they may be derived from any of the new generation methods using tissue culture to grow the virus or express recombinant influenza virus surface antigens. Suitable cell substrates for growing the virus include for example dog kidney cells such as MDCK or cells from a clone of MDCK, MDCK-like cells, monkey kidney cells such as AGMK cells including Vero cells, suitable pig cell lines, or any other mammalian cell type suitable for the production of influenza virus for vaccine purposes. Suitable cell substrates also include human cells e.g. MRC-5 cells or the Per.C6 cell line, and avian cells and cell lines, such as chicken or duck cell lines (e.g. EBx^{®} cell line such as EB14^{®}, EB24^{®} or EB66^{®} derived from chicken or duck embryonic stem cells) are also included. EB66^{®} is particularly preferred. Other suitable insect cells are Sf9 or Hi5. Suitable cell substrates are not limited to cell lines; for example primary cells such as chicken embryo fibroblasts are also included.

The influenza virus antigen preparation may be produced by any of a number of commercially applicable processes, for example the split flu process described in patent no. DD 300 833 and DD 211 444, incorporated herein by reference. Traditionally split flu was produced using a solvent/detergent treatment, such as tri-*n-*butyl phosphate, or diethylether in combination with Tween™ (known as "Tween-ether" splitting) and this process is still used in some production facilities. Other splitting agents now employed include detergents or proteolytic enzymes or bile salts, for example sodium deoxycholate as described in patent no. DD 155 875, incorporated herein by reference. Detergents that can be used as splitting agents include cationic detergents e.g. cetyl trimethyl ammonium bromide (CTAB), other ionic detergents e.g. laurylsulfate, taurodeoxycholate, or non-ionic detergents such as the ones described above including Triton X-100 (for example in a process described in Lina et al, 2000, Biologicals 28, 95-103) and Triton N-101, or combinations of any two or more detergents.

The preparation process for a split vaccine will include a number of different filtration and/or other separation steps such as ultracentrifugation, ultrafiltration, zonal centrifugation and chromatography (e.g. ion exchange) steps in a variety of combinations, and optionally an inactivation step eg with heat, formaldehyde or β-propiolactone or U. V. which may be carried out before or after splitting. The splitting process may be carried out as a batch, continuous or semi-continuous process.

Preferred split flu vaccine antigen preparations according to the invention comprise a residual amount of Tween 80 and/or Triton X-100 remaining from the production process, although these may be added or their concentrations adjusted after preparation of the split antigen. Preferably both Tween 80 and Triton X-100 are present. The preferred ranges for the final concentrations of these non-ionic surfactants in the vaccine dose are:
Tween 80: 0.01 to 1%, more preferably about 0.1% (v/v)
Triton X-100: 0.001 to 0.1 (% w/v), more preferably 0.005 to 0.02% (w/v).

Alternatively the influenza virus antigen preparations according to the invention may be derived from a source other than the live influenza virus, for example the haemagglutinin antigen may be produced recombinantly.

The invention will now be further described in the following examples.

### EXAMPLES

### Example 1- Preparation of influenza virus antigen preparation using α-tocopherol succinate as a stabiliser for a preservative-free vaccine (thiomersal-reduced vaccine)

Monovalent split vaccine was prepared according to the following procedure.

### Preparation of virus inoculum

On the day of inoculation of embryonated eggs a fresh inoculum is prepared by mixing the working seed lot with a phosphate buffered saline containing gentamycin sulphate at 0.5 mg/ml and hydrocortisone at 25 µg/ml. (virus strain-dependent). The virus inoculum is kept at 2-8°C.

### Inoculation of embryonated eggs

Nine to eleven day old embryonated eggs are used for virus replication. Shells are decontaminated. The eggs are inoculated with 0.2 ml of the virus inoculum. The inoculated eggs are incubated at the appropriate temperature (virus strain-dependent) for 48 to 96 hours. At the end of the incubation period, the embryos are killed by cooling and the eggs are stored for 12-60 hours at 2-8°C.

### Harvest

The allantoic fluid from the chilled embryonated eggs is harvested. Usually, 8 to 10 ml of crude allantoic fluid is collected per egg.

### Concentration and purification of whole virus from allantoic fluid

### 1. Clarification

The harvested allantoic fluid is clarified by moderate speed centrifugation (range: 4000 - 14000 g).

### 2. Adsorption step

To obtain a CaHPO₄ gel in the clarified virus pool, 0.5 mol/L Na₂HPO₄ and 0.5mol/L CaCl₂ solutions are added to reach a final concentration of CaHPO₄ of 1.5 g to 3.5 g CaHPO₄/litre depending on the virus strain.

After sedimentation for at last 8 hours, the supernatant is removed and the sediment containing the influenza virus is resolubilised by addition of a 0.26 mol/L EDTA-Na₂ solution, dependent on the amount of CaHPO₄ used.

### 3. Filtration

The resuspended sediment is filtered on a 6µm filter membrane.

### 4. Sucrose gradient centrifugation

The influenza virus is concentrated by isopycnic centrifugation in a linear sucrose gradient (0.55 % (w/v)) containing 100 µg/ml Thiomersal. The flow rate is 8 - 15 litres/hour.

At the end of the centrifugation, the content of the rotor is recovered by four different fractions (the sucrose is measured in a refractometer):

| | |
|---|---|
| - fraction 1 | 55-52% sucrose |
| - fraction 2 | approximately 52-38% sucrose |
| - fraction 3 | 38-20% sucrose* |
| - fraction 4 | 20- 0% sucrose |

| | |
|---|---|
| * virus strain-dependent: fraction 3 can be reduced to 15% sucrose. | |

For further vaccine preparation, only fractions 2 and 3 are used.

Fraction 3 is washed by diafiltration with phosphate buffer in order to reduce the sucrose content to approximately below 6%. The influenza virus present in this diluted fraction is pelleted to remove soluble contaminants.

The pellet is resuspended and thoroughly mixed to obtain a homogeneous suspension. Fraction 2 and the resuspended pellet of fraction 3 are pooled and phosphate buffer is added to obtain a volume of approximately 40 litres. This product is the monovalent whole virus concentrate.

### 5. Sucrose gradient centrifugation with sodium deoxycholate

The monovalent whole influenza virus concentrate is applied to a ENI-Mark II ultracentrifuge. The K3 rotor contains a linear sucrose gradient (0.55 % (w/v)) where a sodium deoxycholate gradient is additionally overlayed. Tween 80 is present during splitting up to 0.1 % (w/v) and Tocopherol succinate is added for B-strain- viruses up to 0.5 mM. The maximal sodium deoxycholate concentration is 0.7-1.5 % (w/v) and is strain dependent. The flow rate is 8 - 15 litres/hour.

At the end of the centrifugation, the content of the rotor is recovered by three different fractions (the sucrose is measured in a refractometer) Fraction 2 is used for further processing. Sucrose content for fraction limits (47-18%) varies according to strains and is fixed after evaluation:

### 6. Sterile filtration

The split virus fraction is filtered on filter membranes ending with a 0.2 µm membrane. Phosphate buffer containing 0.025 % (w/v) Tween 80 and (for B strain viruses) 0.5 mM Tocopherol succinate is used for dilution. The final volume of the filtered fraction 2 is 5 times the original fraction volume.

### 7. Inactivation

The filtered monovalent material is incubated at 22 ± 2°C for at most 84 hours (dependent on the virus strains, this incubation can be shortened). Phosphate buffer containing 0.025% (w/v). Tween 80 is then added in order to reduce the total protein content down to max. 250 µg/ml. For B strain viruses, a phosphate buffered saline containing 0.025% (w/v) Tween 80 and 0.25 mM Tocopherol succinate is applied for dilution to reduce the total protein content down to 250 µg/ml. Formaldehyde is added to a final concentration of 50 µg/ml and the inactivation takes place at 20°C ± 2°C for at least 72 hours.

### 8. Ultrafiltration

The inactivated split virus material is concentrated at least 2 fold in a ultrafiltration unit, equipped with cellulose acetate membranes with 20 kDa MWCO. The Material is subsequently washed with phosphate buffer containing 0.025 % (w/v) Tween 80 and following with phosphate buffered saline containing 0.01 % (w/v) Tween. For B strain virus a phosphate buffered saline containing 0.01% (w/v) Tween 80 and 0.1 mM Tocopherol succinate is used for washing.

### 9. Final sterile filtration

The material after ultrafiltration is filtered on filter membranes ending with a 0.2 µm membrane. Filter membranes are rinsed and the material is diluted if necessary such that the protein concentration does not exceed 500 µg/ml with phosphate buffered saline containing 0.01% (w/v) Tween 80 and (for B strain viruses) 0.1 mM Tocopherol succinate.

### 10. Storage

The monovalent final bulk is stored at 2 - 8°C for a maximum of 18 months.

### Stability

**Table 1. Comparison of time dependent HA content (µg/ml) measured by SRD in monovalent final bulks.**

| Strain | Stabiliser | After production | 4 weeks at 30°C | 6 month at 2-8°C | 12 month at 2-8°C |
|---|---|---|---|---|---|
| | | | | | |
| B/Yamanashi/166/98 | Tocopherylsuccinate (residual mercury 3 µg/ml) | 169 | 139 (82%) | 172 (> 100%) | ND |
| B/Yamanashi/166/98 | Thiomersal (108 µg/ml) | 192 | 160 (83%) | 186 (97%) | 178 (93%) |
| B/Yamanashi/166/98 | None (residual mercury 3 µg/ml) | 191 | 122 (60%) | 175 (92%) | 154 (81%) |
| B/Johannesburg/5/99 | Tocopherylsuccinate (residual mercury 4 µg/ml) | 166 | 183 (> 100%) | 158 (95%) | 179 (> 100%) |
| B/Johannesburg/5/99 | Tocopherylsuccinate | 167 | 179 | 158 | 178 |
| | (residual mercury 4 µg/ml) | | (> 100%) | (95%) | (> 100%) |
| B/Johannesburg/5/99 | Tocopherylsuccinate (residual mercury 3 µg/ml) | 144 | 151 (> 100%) | 130 (90%) | 145 (> 100%) |
| B/Johannesburg/5/99* | Thiomersal | 159 | ND | 172 (> 100%) | 154 (97%) |
| B/Johannesburg/5/99** | None | 169 | 107 (63%) | 153 (90%) | ON |

| | | | | | |
|---|---|---|---|---|---|
| * produced according to licensed FLUARIX™, ** produced according to example 1 without Tocopherylsuccinate, ON: Ongoing, ND not determined | | | | | |

### Example 2 - Preparation of influenza vaccine using α-tocopherol succinate as a stabiliser for a thiomersal-reduced vaccine

Monovalent final bulks of three strains, A/New Caledonia/20/99 (H1N1) IVR-116, A/Panama/2007/99 (H3N2) Resvir-17 and B/Yamanashi/166/98 were produced according to the method described in Example 1.

### Pooling

The appropriate amount of monovalent final bulks were pooled to a final HA-concentration of 30 µg/ml for A/New Caldonia/20/99 (H1N1) IVR-116, A/Panama/2007/99 (H3N2) Resvir-17, respectively and of 39 µg/ml for B/Yamanashi/166/98. Tween 80 and Triton X - 100 were adjusted to 580 µg/ml and 90 µg/ml, respectively. The final volume was adjusted to 31 with phosphate buffered saline. The trivalent pool was filtered ending with 0.8 µm cellulose acetate membrane to obtain the trivalent final bulk. Trivalent final bulk was filled into syringes at least 0.5 mL in each.

**Table 2. Comparison of time dependent HA content measured by SRD in trivalent final bulks which was recovered from syringes.**

| **Vaccine formul.** | **Strain** | **0 months** | **2 months** | **4 months** | **6 months** |
|---|---|---|---|---|---|
| | | | | | |
| Influenza vaccine without stabilizer | A/NCal/20/99 | 33 (32-34) | 32 (31-33) | 36 (34-38) | 31 (30-32) |
| | A/Pan/2007/99 | 29 (27-31) | 31 (28-34) | 34 (32-36) | 32 (31-33) |
| | **B/Yam/166/98** | **36 (34-38)** | **33 (32-34)** | **32 (30-34)** | **31 (29-33)** |
| | | | | | |
| Influenza vaccine containing | A/NCal/20/99 | 31 (30-32) | 32 (31-33) | 36 (34-38) | 32 (31-33) |
| alpha-tocopherol succinate | A/Pan/2007/99 | 33 (30-36) | 33 (30-36) | 36 (35-37) | 33 (31-35) |
| | **B/Yam/166/98** | **37 (35-39)** | **36 (34-38)** | **38 (35-41)** | **36 (33-39)** |

### Example 3 - SRD Method used to measure haemagglutinin content

Glass plates (12.4 - 10.0 cm) are coated with an agarose gel containing a concentration of anti-influenza HA serum that is recommended by NIBSC. After the gel has set, 72 sample wells (3 mm Ø) are punched into the agarose. 10 microliters of appropriate dilutions of the reference and the sample are loaded in the wells. The plates are incubated for 24 hours at room temperature (20 to 25°C) in a moist chamber. After that, the plates are soaked overnight with NaCl-solution and washed briefly in distilled water. The gel is then pressed and dried. When completely dry, the plates are stained on Coomassie Brillant Blue solution for 10 min and destained twice in a mixture of methanol and acetic acid until clearly defined stained zones become visible. After drying the plates, the diameter of the stained zones surrounding antigen wells is measured in two directions at right angles. Alternatively equipment to measure the surface can be used. Dose-response curves of antigen dilutions against the surface are constructed and the results are calculated according to standard slope-ratio assay methods (Finney, D.J. (1952). Statistical Methods in Biological Assay. London: Griffin, Quoted in: Wood, JM, et al (1977). J. Biol. Standard. 5, 237-247).

### Example 4 - Clinical testing of α-tocopherol stabilised influenza vaccine (reduced thiomersal)

Syringes obtained as described in Example 2 are used for clinical testing
H3N2: A/Panama/2007/99 RESVIR-17
H1N1: A/New Caledonia/20/99 (H1N1) IVR-116
B: B/Yamanashi/166/98

**Table 3**

| | **Adults 18-60 years** | | | | | | |
|---|---|---|---|---|---|---|---|
| | | **thio-reduced** | | | **thio-plus** | | |
| | | | | | | | |

| | | **H3N2** | **H1N1** | **B** | **H3N2** | **H1N1** | **B** |
|---|---|---|---|---|---|---|---|
| | | | | | | | |
| **pre**- | GMT | 47 | 41 | 111 | 55 | 37 | 102 |
| **vacc**. | | | | | | | |
| | Titer <10 [%] | 10.3% | 13.8% | 1.7% | 5.3% | 12.3% | 8.8% |
| | | | | | | | |
| | Titer ≥40, SPR [%] | 60.3% | 55.2% | 75.9% | 70.2% | 52.6% | 75.4% |
| | | | | | | | |
| | | | | | | | |
| **post**- | Seroconv. rate [%] | 10.3% | 13.8% | 1.7% | 5.3% | 12.3% | 8.8% |
| **vacc**. | | | | | | | |
| | Significant Increase in antibody titer [%] | 58.6% | 74.1% | 58.6% | 63.2% | 73.7% | 52.6% |
| | | | | | | | |
| | | | | | | | |
| | Seroconversions [%] | **58**.**6**% | **74**.**1**% | **58**.**6**% | **63**.**2**% | **73.7%** | **52**.**6**% |
| | | | | | | | |
| | GMT | 328 | 525 | 766 | 324 | 359 | 588 |
| | | | | | | | |
| | Fold GMT | **7**.**3** | **13**.**0** | **6**.**9** | **5**.**9** | **9**.**8** | **5**.**9** |
| | | | | | | | |
| | Titer ≥40, SPR [%] | **100**.**0**% | **100**.**0**% | **100**.**0**% | **100**.**0**% | **100**.**0**% | **100**.**0**% |

| | | | | | | | |
|---|---|---|---|---|---|---|---|
| n.d. = C.I. for proportion p=n/N is not defined, because p*(1-p)*N < 9 n/N = responders (n) as part of number of subjects of the (sub)population (N), i.e. seroconversions or significant increase, see also: CPMAP/BWP/214/96 12 March 1997, p.17ff GMT = geometric mean titer, reciprocal 95% C.I. = 95% confidence interval, SPR = Seroprotection rate: proportion of subjects with a protective titer pre- or postvaccination ≥40 titer = HI-antibody titer Seroconversion rate = proportion of subjects with antibody increase from <10 prevaccination to ≥40 postvaccination fold GMT = fold increase of GMT Significant increase = proportion of subjects with an antibody titer ≥10 prevaccination and 4-fold antibody increase postvaccination (two steps of titer) req. = EU requirement Seroconversions = neg to pos or g.e. 4-fold (neg: titer <10, pos: titer ≥40) = proportion of subjects with either seroconversion (<10 to ≥40) or significant increase. | | | | | | | |

Results show that the vaccine is able to offer equivalent protection to vaccines containing thiomersal as a preservative.

### Example 5a - Preparation of influenza virus antigens preparation using α-tocopherol succinate as a stabiliser for a thiomersal-free vaccine

Monovalent split vaccine was prepared according to the following procedure.

### Preparation of virus inoculum

On the day of inoculation of embryonated eggs a fresh inoculum is prepared by mixing the working seed lot with a phosphate buffered saline containing gentamycin sulphate at 0.5 mg/ml and hydrocortisone at 25 µg/ml. (virus strain-dependent). The virus inoculum is kept at 2-8°C.

### Inoculation of embryonated eggs

Nine to eleven day old embryonated eggs are used for virus replication. Shells are decontaminated. The eggs are inoculated with 0.2 ml of the virus inoculum. 60,000 inoculated eggs are incubated at the appropriate temperature (virus strain-dependent) for 48 to 96 hours. At the end of the incubation period, the embryos are killed by cooling and the eggs are stored for 12-60 hours at 2-8°C.

### Harvest

The allantoic fluid from the chilled embryonated eggs is harvested. Usually, 8 to 10 ml of crude allantoic fluid is collected per egg.

### Concentration and purification of whole virus from allantoic fluid

### Clarification

The harvested allantoic fluid is clarified by moderate speed centrifugation (range: 4000 - 14000 g).

### Precipitation step

Saturated ammonium sulfate solution is added to the clarified virus pool to reach a final ammonium sulfate concentration of 0.5 mol/L. After sedimentation for at least 1 hour, the precipitate is removed by filtration on depth filters (typically 0.5 µm)

### Filtration

The clarified crude whole virus bulk is filtered on filter membranes ending with a validated sterile membrane (typically 0.2 µm).

### Ultrafiltration

The sterile filtered crude monovalent whole virus bulk is concentrated on a cassettes equipped with 1000 kDa MWCO BIOMAX™ membrane at least 6 fold. The concentrated retentate is washed with phosphate buffered saline at least 1.8 times.

### Sucrose gradient centrifugation

The influenza virus is concentrated by isopycnic centrifugation in a linear sucrose gradient (0.55 % (w/v)). The flow rate is 8 - 15 litres/hour.

At the end of the centrifugation, the content of the rotor is recovered by four different fractions (the sucrose is measured in a refractometer):

| | |
|---|---|
| - fraction 1 | 55-52% sucrose |
| - fraction 2 | approximately 52-38% sucrose |
| - fraction 3 | 38-20% sucrose* |
| - fraction 4 | 20- 0% sucrose |

| | |
|---|---|
| * virus strain-dependent: fraction 3 can be reduced to 15% sucrose. | |

For further vaccine preparation, either only fractions 2 is used or fraction 2 together with a further purified fraction 3 are used.

Fraction 3 is washed by diafiltration with phosphate buffer in order to reduce the sucrose content to approximately below 6%. Optionally this step may be omitted. The influenza virus present in this diluted fraction is pelleted to remove soluble contaminants.

The pellet is resuspended and thoroughly mixed to obtain a homogeneous suspension. Fraction 2 and the resuspended pellet of fraction 3 are pooled and phosphate buffer is added to obtain a volume of approximately 40 litres. This product is the monovalent whole virus concentrate.

### Sucrose gradient centrifugation with sodium deoxycholate

The monovalent whole influenza virus concentrate is applied to a ENI-Mark II ultracentrifuge. The K3 rotor contains a linear sucrose gradient (0.55 % (w/v)) where a sodium deoxycholate gradient is additionally overlayed. Tween 80 is present during splitting up to 0.1 % (w/v) and Tocopherylsuccinate is added for B-strain viruses up to 0.5 mM. The maximal sodium deoxycholate concentration is 0.7-1.5 % (w/v) and is strain dependent. The flow rate is 8 - 15 litres/hour.

At the end of the centrifugation, the content of the rotor is recovered by three different fractions (the sucrose is measured in a refractometer) Fraction 2 is used for further processing. Sucrose content for fraction limits (47-18%) varies according to strains and is fixed after evaluation:

### Sterile filtration

The split virus fraction is filtered on filter membranes ending with a 0.2 µm membrane. Phosphate buffer containing 0.025 % (w/v) Tween 80 and (for B strains) 0.5 mM Tocopherylsuccinate is used for dilution. The final volume of the filtered fraction 2 is 5 times the original fraction volume.

### Inactivation

The filtered monovalent material is incubated at 22 ± 2°C for at most 84 hours (dependent on the virus strains, this incubation can be shortened). Phosphate buffer containing 0.025% (w/v) Tween 80 is then added in order to reduce the total protein content down to max. 450 µg/ml. For B-strains a phosphate buffered saline containing 0.025% (w/v) Tween 80 and 0.25 mM Tocopherylsuccinate is applied for dilution to reduce the total protein content down to 450 µg/ml. Formaldehyde is added to a final concentration of 100 µg/ml and the inactivation takes place at 20°C ± 2°C for at least 72 hours.

### Ultrafiltration

The inactivated split virus material is concentrated at least 2 fold in a ultrafiltration unit, equipped with cellulose acetate membranes with 20 kDa MWCO. The Material is subsequently washed with phosphate buffer containing 0.025 % (w/v) Tween 80 and following with phosphate buffered saline containing 0.01 % (w/v) Tween. For B-strain viruses a phosphate buffered saline containing 0.01% (w/v) Tween 80 and 0.1 mM Tocopherylsuccinate is used for washing.

### Final sterile filtration

The material after ultrafiltration is filtered on filter membranes ending with a 0.2 µm membrane. Filter membranes are rinsed and the material is diluted if necessary that the protein concentration does not exceed 500 µg/ml with phosphate buffered saline containing 0.01 % (w/v) Tween 80 and, specific for B strains, 0.1 mM Tocopherylsuccinate.

### Storage

The monovalent final bulk is stored at 2 - 8°C for a maximum of 18 months.

### Stability

**Table 4. Comparison of time dependent HA content (µg/ml) measured by SRD in monovalent final bulks.**

| Strain | Stabiliser | After production | 4 weeks at 30°C | 6 month at 2-8°C |
|---|---|---|---|---|
| | | | | |
| B/Johannesburg/5/99 | Tocopherol succinate | 214 | 196 (92%) | 206 (96%) |
| B/Johannesburg/5/99** | None | 169 | 107 (63%) | 153 (90%) |

| | | | | |
|---|---|---|---|---|
| ** produced according to example 1 without Tocopherylsuccinate. | | | | |

### Example 5b - Preparation of influenza virus antigen preparation using α-tocopherol succinate as a stabiliser for a thiomersal-free vaccine

A preferred variation of the method described in Example 5a is as follows:
Harvesting of the whole virus is followed by the precipitation step (ammonium sulfate precipitation). This is followed by the clarification step where the fluid is clarified by moderate speed centrifugation (range 4000 - 14000 g). Thus the order of the precipitation and clarification steps is reversed compared to Example 5a.

Sterile filtration, ultrafiltration and ultracentrifugation (sucrose gradient centrifugation) steps follow as for Example 5a. However, there is no need for reprocessing step of the fractions resulting from the ultracentrifugation step.

The remaining steps in the process are as described in Example 5a.

Thus, the summarised process in this example is as follows:
Harvest
Precipitation (ammonium sulfate)
Clarification
Sterile filtration
Ultrafiltration
Ultracentrifugation
Splitting (preferably sodium deoxycholate)
Sterile filtration
Inactivation
Ultrafiltration
Final sterile filtration

Another preferred variation of Example 5a involves a prefiltration step before the first sterile filtration. This uses a membrane which does not sterile filter but which enables the removal of contaminants e.g. albumin prior to sterile filtration. This can result in a better yield. A suitable membrane for prefiltration is about 0.8 µm to about 1.8 µm, for example 1.2 µm. The prefiltration step can be used in the scheme of Example 5a or Example 5b.

### Example 6 - Preparation of influenza vaccine using α-tocopherol succinate as a stabiliser for a thiomersal-free vaccine

Monovalent final bulks of three strains, A/New Caldonia/20/99 (H1N1) IVR-116, A/Panama/2007/99 (H3N2) Resvir-17 and B/Yamanashi/166/98 were produced according to the method described in Example 5.

### Pooling

The appropriate amount of monovalent final bulks were pooled to a final HA-concentration of 30 µg/ml for A/New Caldonia/20/99 (H1N1) IVR-116, A/Panama/2007/99 (H3N2) Resvir-17, respectively and of 36 µg/ml for B/Johannesburg/5/97. Tween 80 and Triton X - 100 were adjusted to 580 µg/ml and 90 µg/ml, respectively. The final volume was adjusted to 31 with phosphate buffered saline. The trivalent pool was filtered ending with 0.8 µm cellulose acetate membrane to obtain the trivalent final bulk.Trivalent final bulk was filled into syringes at least 0.5 mL in each.

**Table 5. Comparison of time dependent HA content (µg/ml) measured by SRD in trivalent final bulks.**

| **Vaccine formul.** | **Strain** | **0 months** | **4 weeks at 30°C** | **6 months at 2-8°C** |
|---|---|---|---|---|
| | | | | |
| Influenza vaccine without stabilizer | A/NCal/20/99 | 31 | 32 | 30 |
| | A/Pan/2007/99 | 31 | 34 | 33 |
| | **B/Joh/5/99*** | **35** | **25** | **31** |
| | | | | |
| Influenza vaccine containing | A/NCal/20/99 | 34 | 35 | 34 |
| alpha-tocopherol succinate | A/Pan/2007/99 | 33 | 33 | 34 |
| | **B/Joh/5/99**** | **29** | **25** | **28** |

| | | | | |
|---|---|---|---|---|
| *Formulation was based on target concentration of 39 µg/ml. **Formulation was based on target concentration of 34 µg/ml. | | | | |

### Example 7 - Preparation of influenza virus antigen preparation using sodium lauryl sulfate as a stabiliser for a preservative-free vaccine (thiomersal-reduced vaccine)

### Monovalent whole virus concentrate of B/Johannesburg/5/99 was obtained as described in Example 1.

### Sucrose gradient centrifugation with sodium deoxycholate

The monovalent whole influenza virus concentrate is applied to a ENI-Mark II ultracentrifuge. The K3 rotor contains a linear sucrose gradient (0.55 % (w/v)) where a sodium deoxycholate gradient is additionally overlayed. Tween 80 is present during splitting up to 0.1 % (w/v). The maximal sodium deoxycholate concentration is 0.7-1.5 % (w/v) and is strain dependent. The flow rate is 8 - 15 litres/hour.

At the end of the centrifugation, the content of the rotor is recovered by three different fractions (the sucrose is measured in a refractometer) Fraction 2 is used for further processing. Sucrose content for fraction limits (47-18%) varies according to strains and is fixed after evaluation:

### Sterile filtration

A sample of fraction 2 of 10 ml was taken for further processing. The split virus fraction is filtered on filter membranes ending with a 0.2 µm membrane. Phosphate buffer containing 0.025 % (w/v) Tween 80 and 0.5 mM sodium lauryl sulfate is used for dilution. The final volume of the filtered fraction 2 is 5 times the original fraction volume.

### Inactivation

The filtered monovalent material is incubated at 22 ± 2°C for at most 84 hours (dependent on the virus strains, this incubation can be shortened). Phosphate buffered saline containing 0.025% (w/v) Tween 80 and 0.5 mM sodium laurylsulfate is then added in order to reduce the total protein content down to max. 250 µg/ml. Formaldehyde is added to a final concentration of 50 µg/ml and the inactivation takes place at 20°C ± 2°C for at least 72 hours.

### Ultrafiltration

The inactivated split virus material is concentrated at least 2 fold in a ultrafiltration unit, equipped with cellulose acetate membranes with 20 kDa MWCO. The Material is subsequently washed with 4 volumes phosphate buffered saline containing 0.01 % (w/v) Tween and 0.5 mM sodium lauryl sulfate.

### Final sterile filtration

The material after ultrafiltration is filtered on filter membranes ending with a 0.2 µm membrane. Filter membranes are rinsed and the material is diluted if necessary that the protein concentration does not exceed 500 µg/ml with phosphate buffered saline containing 0.01% (w/v) Tween 80 and 0.5 mM sodium lauryl sulfate.

### Storage

The monovalent final bulk is stored at 2 - 8°C.

**Table 7. Comparison of time dependent HA content measured by SRD in monovalent final bulks.**

| | stabiliser | After production | 4 weeks at 30°C |
|---|---|---|---|
| B/Johannesburg/5/99 | None* | 182 | 139 (77 %) |
| B/Johannesburg/5/99 | Sodium lauryl sulfate | 288 | 264 (92 %) |

| | | | |
|---|---|---|---|
| * produced according to Example 7 without addition of sodium lauryl sulfate | | | |

### Example 8 - Preparation of influenza virus antigen preparation using Plantacare or Laureth-9 as a stabiliser for a preservative-free vaccine (thiomersal-reduced vaccine)

### Monovalent whole virus concentrate of B/Yamanashi/166/98 was obtained as described in Example 1.

### Fragmentation

The monovalent whole influenza virus concentrate is diluted to a protein concentration of 1,000 µg/ml with phosphate buffered saline pH 7.4. Either Plantacare® 2000 UP or Laureth-9 is added to a final concentration of 1% (w/v). The material is slightly mixed for 30 min. Then the material is overlayed on a sucrose cushion 15% (w/w) in a bucket. Ultracentrifugation in a Beckman swing out rotor SW 28 is performed for 2 h at 25,000 rpm and 20°C.

### Sterile filtration

A supernatant was taken for further processing. The split virus fraction is filtered on filter membranes ending with a 0.2 µm membrane.

### Inactivation

Phosphate buffered saline is added if necessary in order to reduce the total protein content down to max. 500 µg/ml. Formaldehyde is added to a final concentration of 100 µg/ml and the inactivation takes place at 20°C ± 2°C for at least 6 days.

### Ultrafiltration

Tween 80 and Triton X 100 is adjusted in the inactivated material to 0.15% and 0.02 % respectively. The inactivated split virus material is concentrated at least 2 fold in a ultrafiltration unit, equipped with cellulose acetate membranes with 30 kDa MWCO. The Material is subsequently washed with 4 volumes phosphate buffered saline.

### Final sterile filtration

The material after ultrafiltration is filtered on filter membranes ending with a 0.2 µm membrane. Filter membranes are rinsed and the material is diluted that the protein concentration does not exceed 500 µg/ml with phosphate buffered saline

### Storage

The monovalent final bulk is stored at 2 - 8°C.

**Table 8. Comparison of time dependent HA content measured by SRD in monovalent final bulks.**

| | stabiliser | After production | 4 weeks at 30°C |
|---|---|---|---|
| B/Yamanashi/166/98 | None | 143 | 98 (68 %) |
| B/Yamanashi/166/98 | Plantacare® 2000 UP | 476 | 477(100%) |
| B/Yamanashi/166/98 | Laureth-9 | 468 | 494 (> 100%) |

### Example 9 - Clinical testing of α-tocopherol stabilised influenza vaccine (reduced thiomersal) in the elderly via ID and IM administration

### A Preparation of influenza virus antigen preparation

Monovalent split vaccine was prepared according to the following procedure.

### Preparation of virus inoculum

On the day of inoculation of embryonated eggs a fresh inoculum is prepared by mixing the working seed lot with a phosphate buffered saline containing gentamycin sulphate at 0.5 mg/ml and hydrocortisone at 25 µg/ml. (virus strain-dependent). The virus inoculum is kept at 2-8°C.

### Inoculation of embryonated eggs

Nine to eleven day old embryonated eggs are used for virus replication. Shells are decontaminated. The eggs are inoculated with 0.2 ml of the virus inoculum. The inoculated eggs are incubated at the appropriate temperature (virus strain-dependent) for 48 to 96 hours. At the end of the incubation period, the embryos are killed by cooling and the eggs are stored for 12-60 hours at 2-8°C.

### Harvest

The allantoic fluid from the chilled embryonated eggs is harvested. Usually, 8 to 10 ml of crude allantoic fluid is collected per egg.

### Concentration and purification of whole virus from allantoic fluid

### 1. Clarification

The harvested allantoic fluid is clarified by moderate speed centrifugation (range: 4000 - 14000 g).

### 2. Adsorption step

To obtain a CaHPO₄ gel in the clarified virus pool, 0.5 mol/L Na₂HPO₄ and 0.5mol/L CaCl₂ solutions are added to reach a final concentration of CaHPO₄ of 1.5 g to 3.5 g CaHPO₄/litre depending on the virus strain.

After sedimentation for at last 8 hours, the supernatant is removed and the sediment containing the influenza virus is resolubilised by addition of a 0.26 mol/L EDTA-Na₂ solution, dependent on the amount of CaHPO₄ used.

### 3. Filtration

The resuspended sediment is filtered on a 6µm filter membrane.

### 4. Sucrose gradient centrifugation

The influenza virus is concentrated by isopycnic centrifugation in a linear sucrose gradient (0.55 % (w/v)) containing 100 µg/ml Thiomersal. The flow rate is 8 - 15 litres/hour.

At the end of the centrifugation, the content of the rotor is recovered by four different fractions (the sucrose is measured in a refractometer):

| | |
|---|---|
| - fraction 1 | 55-52% sucrose |
| - fraction 2 | approximately 52-38% sucrose |
| - fraction 3 | 38-20% sucrose* |
| - fraction 4 | 20- 0% sucrose |

| | |
|---|---|
| * virus strain-dependent: fraction 3 can be reduced to 15% sucrose. | |

For further vaccine preparation, only fractions 2 and 3 are used.

Fraction 3 is washed by diafiltration with phosphate buffer in order to reduce the sucrose content to approximately below 6%. The influenza virus present in this diluted fraction is pelleted to remove soluble contaminants.

The pellet is resuspended and thoroughly mixed to obtain a homogeneous suspension. Fraction 2 and the resuspended pellet of fraction 3 are pooled and phosphate buffer is added to obtain a volume of approximately 40 litres, a volume appropriate for 120,000 eggs/batch. This product is the monovalent whole virus concentrate.

### 5. Sucrose gradient centrifugation with sodium deoxycholate

The monovalent whole influenza virus concentrate is applied to a ENI-Mark II ultracentrifuge. The K3 rotor contains a linear sucrose gradient (0.55 % (w/v)) where a sodium deoxycholate gradient is additionally overlayed. Tween 80 is present during splitting up to 0.1 % (w/v) and Tocopherol succinate is added for B-strain- viruses up to 0.5 mM. The maximal sodium deoxycholate concentration is 0.7-1.5 % (w/v) and is strain dependent. The flow rate is 8 - 15 litres/hour.

At the end of the centrifugation, the content of the rotor is recovered by three different fractions (the sucrose is measured in a refractometer) Fraction 2 is used for further processing. Sucrose content for fraction limits (47-18%) varies according to strains and is fixed after evaluation:

### 6. Sterile filtration

The split virus fraction is filtered on filter membranes ending with a 0.2 µm membrane. Phosphate buffer containing 0.025 % (w/v) Tween 80 and (for B strain viruses) 0.5 mM Tocopherol succinate is used for dilution. The final volume of the filtered fraction 2 is 5 times the original fraction volume.

### 7. Inactivation

The filtered monovalent material is incubated at 22 ± 2°C for at most 84 hours (dependent on the virus strains, this incubation can be shortened). Phosphate buffer containing 0.025% (w/v). Tween 80 is then added in order to reduce the total protein content down to max. 250 µg/ml. For B strain viruses, a phosphate buffered saline containing 0.025% (w/v) Tween 80 and 0.25 mM Tocopherol succinate is applied for dilution to reduce the total protein content down to 250 µg/ml. Formaldehyde is added to a final concentration of 50 µg/ml and the inactivation takes place at 20°C ± 2°C for at least 72 hours.

### 8. Ultrafiltration

The inactivated split virus material is concentrated at least 2 fold in a ultrafiltration unit, equipped with cellulose acetate membranes with 20 kDa MWCO. The Material is subsequently washed with phosphate buffer containing 0.025 % (w/v) Tween 80 and following with phosphate buffered saline containing 0.01 % (w/v) Tween. For B strain virus a phosphate buffered saline containing 0.01% (w/v) Tween 80 and 0.1 mM Tocopherol succinate is used for washing.

### 9. Final sterile filtration

The material after ultrafiltration is filtered on filter membranes ending with a 0.2 µm membrane. Filter membranes are rinsed and the material is diluted if necessary such that the protein concentration does not exceed 1,000 µg/ml but haemagglutinin concentration exeeds 180 µg/ml with phosphate buffered saline containing 0.01% (w/v) Tween 80 and (for B strain viruses) 0.1 mM Tocopherol succinate.

### 10. Storage

The monovalent final bulk is stored at 2 - 8°C for a maximum of 18 months.

### B Preparation of influenza vaccine

Monovalent final bulks of three strains, A/New Caldonia/20/99 (H1N1) IVR-116, A/Panama/2007/99 (H3N2) Resvir-17 and B/Johannesburg/5/99 were produced according to the method described in part A above.

### Pooling

The appropriate amount of monovalent final bulks were pooled to a final HA-concentration of 60 µg/ml for A/New Caldonia/20/99 (H1N1) IVR-116, A/Panama/2007/99 (H3N2) Resvir-17, respectively and of 68 µg/ml for B/Johannesburg/5/99. Tween 80, Triton X -100 and Tocopherol succinate were adjusted to 1,000 µg/ml, 110 µg/ml and 160 µg/ml, respectively. The final volume was adjusted to 31 with phosphate buffered saline. The trivalent pool was filtered ending with 0.8 µm cellulose acetate membrane to obtain the trivalent final bulk. Trivalent final bulk was filled into syringes at least 0.165 mL in each.

### Vaccine administration

The vaccine was supplied in pre-filled syringes and was administered intradermally in the deltoid region. The intradermal (ID) needle was as described in EP1092444, having a skin penetration limiter to ensure proper intradermal injection. Since formation of a wheal (papule) at the injection site demonstrates the good quality of ID administration, the investigator with the subject measured the exact size of the wheal 30 minutes after vaccination.

One dose (100 µl) contained the following components:

| HEMAGGLUTININ FROM THREE INFLUENZA STRAINS | | | |
|---|---|---|---|
| | A/NEW CALEDONIA/20/99 | : | 6.0 µg |
| | A/PANAMA/2007/99 | : | 6.0 µg |
| | B/JOHANNESBURG 5/99 | : | 6.0 µg |
| THIOMERSAL PRESERVATIVE | | : | 0.4 µg - 0.8 µg |

B The above vaccine was compared a standard trivalent split influenza vaccine: Fluarix™. The Fluarix vaccine was supplied in pre-filled syringes and was administered intramuscularly in the deltoid muscle. A needle of at least 2.5 cm / 1 inch in length (23 gauge) was used to ensure proper intramuscular injection.

One dose (0.5 ml) contained the following components:

| HEMAGGLUTININ FROM THREE INFLUENZA STRAINS | | | |
|---|---|---|---|
| | ANEW CALEDONIA/20/99 | : | 15.0 µg |
| | A/PANAMA/2007/99 | : | 15.0 µg |
| | B/JOHANNESBURG 5/99 | : | 15.0 µg |
| THIOMERSAL PRESERVATIVE | | : | 50.0 µg |

### Results

The mean age of the total cohort at the time of vaccine administration was 70.4 ± 6.2 years Standard Deviation (S.D.), the female/male ratio was 1.7:1.

| **Immunogenicity results:** Analysis of derived immunogenicity variables was as follows: | | | | | | | | |
|---|---|---|---|---|---|---|---|---|
| **Variable** | | **Flu-red ID (N = 65)** | | | **Fluarix™ IM (N = 65)** | | | |
| **GMT** | | **GMT** | **LL** | **UL** | **GMT** | **LL** | **UL** | |
| A/NEW CALEDONIA | PRE | 99.5 | 76.9 | 128.7 | 90.0 | 70.1 | 115.7 | |
| | POST | 165.1 | 129.2 | 211.0 | 174.3 | 133.3 | 227.9 | |
| A/PANAMA | PRE | 75.5 | 54.7 | 104.2 | 69.2 | 51.9 | 92.4 | |
| | POST | 128.6 | 99.1 | 166.8 | 164.3 | 126.0 | 214.1 | |
| B/JOHANNESBURG | PRE | 236.0 | 187.7 | 296.8 | 222.6 | 176.9 | 280.2 | |
| | POST | 341.2 | 276.0 | 421.7 | 402.4 | 312.1 | 518.9 | |

| **Seroconversion rate** | | **%** | **LL** | **UL** | **%** | **LL** | **UL** | |
|---|---|---|---|---|---|---|---|---|
| A/NEW CALEDONIA | | 15.4 | 7.6 | 26.5 | 18.5 | 9.9 | 30.0 | |
| A/PANAMA | | 20.0 | 11.1 | 31.8 | 29.2 | 18.6 | 41.8 | |
| B/JOHANNESBURG | | 9.2 | 3.5 | 19.0 | 16.9 | 8.8 | 28.3 | |

| **Conversion factor** | | **GMR** | **LL** | **UL** | **GMR** | **LL** | **UL** | |
|---|---|---|---|---|---|---|---|---|
| A/NEW CALEDONIA | | 1.7 | 1.4 | 2.0 | 1.9 | 1.6 | 2.3 | |
| A/PANAMA | | 1.7 | 1.4 | 2.1 | 2.4 | 1.9 | 3.0 | |
| B/JOHANNESBURG | | 1.4 | 1.2 | 1.7 | 1.8 | 1.5 | 2.1 | |

| **Seroprotection rate** | | **%** | **LL** | **UL** | **%** | **LL** | **UL** | |
|---|---|---|---|---|---|---|---|---|
| A/NEW CALEDONIA | PRE | 87.7 | 77.2 | 94.5 | 90.8 | 81.0 | 96.5 | |
| | POST | 92.3 | 83.0 | 97.5 | 96.9 | 89.3 | 99.6 | |
| A/PANAMA | PRE | 75.4 | 63.1 | 85.2 | 81.5 | 70.0 | 90.1 | |
| | POST | 90.8 | 81.0 | 96.5 | 93.8 | 85.0 | 98.3 | |
| B/JOHANNESBURG | PRE | 98.5 | 91.7 | 100.0 | 96.9 | 89.3 | 99.6 | |
| | POST | 100.0 | 94.5 | 100.0 | 98.5 | 91.7 | 100.0 | |
| N: number of subjects with available results; %: percentage of subjects within the given parameter; | | | | | | | | |
| LL/UL: lower and upper limit of 95% CI; Pre: at the time of vaccine administration; Post: 21 days after the vaccine dose | | | | | | | | |

Injection site pain, reported by 10/65 (15.4%) vaccinees, was the most common symptom following IM administration of Fluarix™. In the ID group, pain was reported by 3/65 (4.6%) vaccinees. This difference was statistically significant (p=0.038; Fisher exact test). Accordingly the ID delivery of a thiomersal reduced product is preferred.

### Conclusions

Both ID and IM administration of a thio-reduced flu vaccine in an elderly population can provide 100% seroprotection.

A comparable response to vaccination in terms of geometric mean titers, seroprotection rates, seroconversion rates and conversion factors was found in IM and ID vaccinated individuals where the ID group received 2.5-fold less antigen.
There was no discernible difference in the overall incidence of vaccine-related solicited/unsolicited systemic symptoms in the two treatment groups.

### Example 10 - Intradermal delivery of a thiomersal-reduced influenza vaccine

Immunogenicity of the thiomersal reduced split influenza vaccine prepared as described in Example 9 (except that the pooling was done independently and the vaccine was not filled into syringes) was assessed by ID delivery in guinea pigs using a standard needle.

Groups of 5 animals each were primed intranasally with whole inactivated trivalent influenza virus containing 5µg of each HA in a total volume of 200 µl. Twenty-eight days after priming the animals were vaccinated by either the intradermal or intramuscular routes. Intradermal doses containing 0.1, 0.3, or 1.0 µg trivalent thiomersal-reduced split Flu in 0.1 ml were administered in the back of the guinea pig using a standard needle An intramuscular dose of 1.0 µg trivalent thiomersal-reduced split Flu was administered in the hind leg of the guinea pig in a volume of 0.1 ml. The groups were as follows:
- Group 1- 0.1 µg trivalent thiomersal-reduced split Flu ID;
- Group 2 - 0.3 µg trivalent thiomersal-reduced split Flu UD;
- Group 3 - 1.0 µg trivalent thiomersal-reduced split Flu ID
- Group 4 - 1.0 µg trivalent thiomersal-reduced split Flu IM

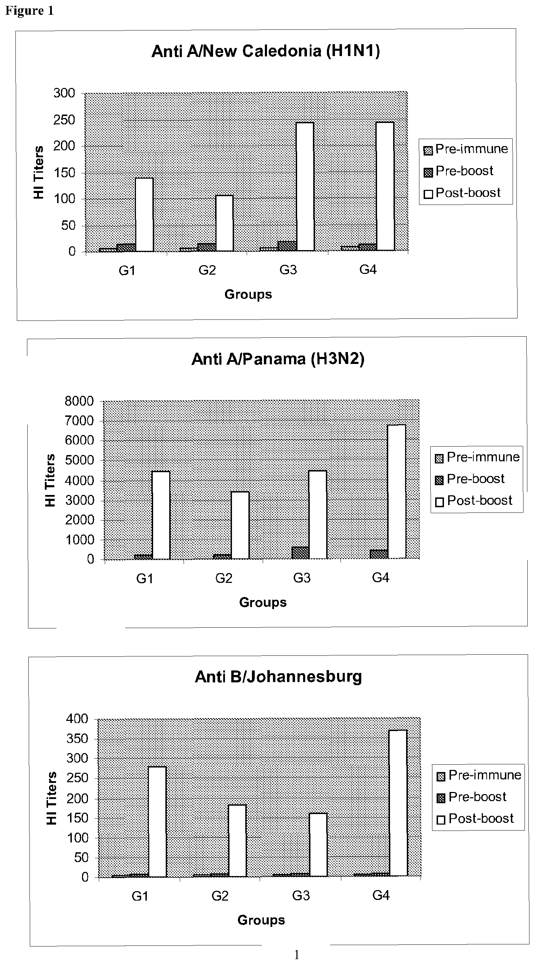

Fourteen days after vaccination the animals were bled and the antibody titers induced by the vaccination were assessed using a standard hemagglutination inhibition assay (HI). The results are shown in Figure 1. Strong HI responses to all three strains were induced by vaccination. No clear dose response was noted suggesting that very low doses of thiomersal-reduced antigen can still induce very potent HI antibody responses when administered by the ID route. There was no significant difference between the HI titers induced by ID or IM vaccination. Thus, the results obtained in guinea pigs confirmed that the thimerosal-reduced trivalent split influenza antigens induce similar levels of HI antibodies in animals when delivered by the ID route compared to the IM route.

### Example 11- Intradermal delivery of a thiomersal-reduced, adjuvanted influenza vaccine

### Protocol

Guinea pigs were primed on Day 0 with 5 µg trivalent whole inactivated Flu virus in 200 µl, intranasally.

Vaccination - Day 28 - Vaccine containing 0.1 µg HA per strain trivalent split Flu prepared as described in Example 9 (except that the pooling step resulted in a final concentration for each antigen of 1.0 µg/ml to give a dose of 0.1 µg in 100 µl compared to 60 µg/ml in Example 9). The final trivalent formulation was administered intradermally using tuberculin syringes, either adjuvanted or unadjuvanted, in 100 µl.

### Bleeding - Day 42.

The effect of adjuvantation was assessed by measuring antibody responses by HI assay (day 0, 28, 42).

All ID experiments were carried out using a standard needle.

### Results

G1-G5 refer to 5 groups of guinea pigs, 5 per group.

| | |
|---|---|
| G1 | Split trivalent thiomersal reduced 0.1µg |
| G2 | Split trivalent thio red 0.1µg + 3D-MPL 50flg |
| G3 | Split trivalent thio red 0.1µg + 3D-MPL 10µg |
| G4 | Split trivalent thio red 0.1µg + 3D-MPLin 50µg + QS21 50µg |
| G5 | Split trivalent thio red 0.1µg + 3D-MPLin 10µg + QS21 10µg |

Note 3D-MPLin + QS21 refers to an adjuvant formulation which comprises a unilamellar vesicle comprising cholesterol, having a lipid bilayer comprising dioleoyl phosphatidyl choline, wherein the QS21 and the 3D-MPL are associated with, or embedded within, the lipid bilayer. Such adjuvant formulations are described in EP 0 822 831 B, the disclosure of which is incorporated herein by reference.

HI Titres anti-A/New Caledonia/20/99

| **NC** | **Pre-immun** | **Pre-boost** | **Post-boost** |
|---|---|---|---|
| G1 | **5** | **10** | **92** |
| G2 | **5** | **10** | **70** |
| G3 | **5** | **11** | **121** |
| G4 | **7** | **9** | **368** |
| G5 | **5** | **10** | **243** |

HI Titres anti-A/Panama/2007/99

| **P** | **Pre-immun** | **Pre-boost** | **Post-boost** |
|---|---|---|---|
| G1 | **5** | **485** | **7760** |
| G2 | **5** | **279** | **7760** |
| G3 | **5** | **485** | **8914** |
| G4 | **7** | **485** | **47051** |
| G5 | **5** | **320** | **17829** |

HI Titres anti-B/Johannesburg/5/99

| **J** | **Pre-immun** | **Pre-boost** | **Post-boost** |
|---|---|---|---|
| G1 | **5** | **23** | **184** |
| G2 | **5** | **11** | **121** |
| G3 | **5** | **11** | **70** |
| G4 | **6** | **15** | **557** |
| G5 | **5** | **13** | **320** |

Thus, whether adjuvanted or unadjuvanted the thiomersal-reduced trivalent split Flu antigen is a potent immunogen and capable of inducing strong HI responses when administered by the ID or IM route. These responses appear to be at least as potent as the responses induced by the standard Fluarix preparation.

### Example 12 - Comparison of thiomersal-containing and thiomersal-free vaccine delivered intradermally in pigs.

In order to assess the immunogenicity of the split Flu vaccine (plus and minus thiomersal) administered by the ID route the primed pig model was used. As the vast majority of the population has experienced at least one infection with influenza an influenza vaccine must be able to boost a pre-existing immune response. Therefore animals are primed in an effort to best simulate the human situation.

In this experiment 4 week old pigs were primed by the intranasal route. Six groups of five animals each were primed as follows:
Group 1 - two primings of trivalent whole inactivated virus (50 µg each HA) at day 0 and 14; Group 2 - two primings of trivalent whole inactivated virus (50 µg each HA) at day 0 and 14; Group 3 - single priming with trivalent whole inactivated virus (50 µg each HA) at day 0; Group 4 - two primings of trivalent whole inactivated virus (25 µg each HA) at day 0 and 14; Group 5 - single priming of trivalent whole inactivated virus (25 µg each HA) at day 0; Group 6 - two primings of trivalent whole inactivated virus (12.5 µg each HA) at day 0 and 14.

On day 28 post final priming, the animals were vaccinated with 3 µg each HA trivalent split antigen (strains A/New Caledonia H1N1, A/Panama H3N2, and B/Johannesburg) in 100 µl by the ID route. Group 1 received standard Fluarix™ containing thiomersal preservative as vaccine antigen. All other groups received the preservative-free antigen.

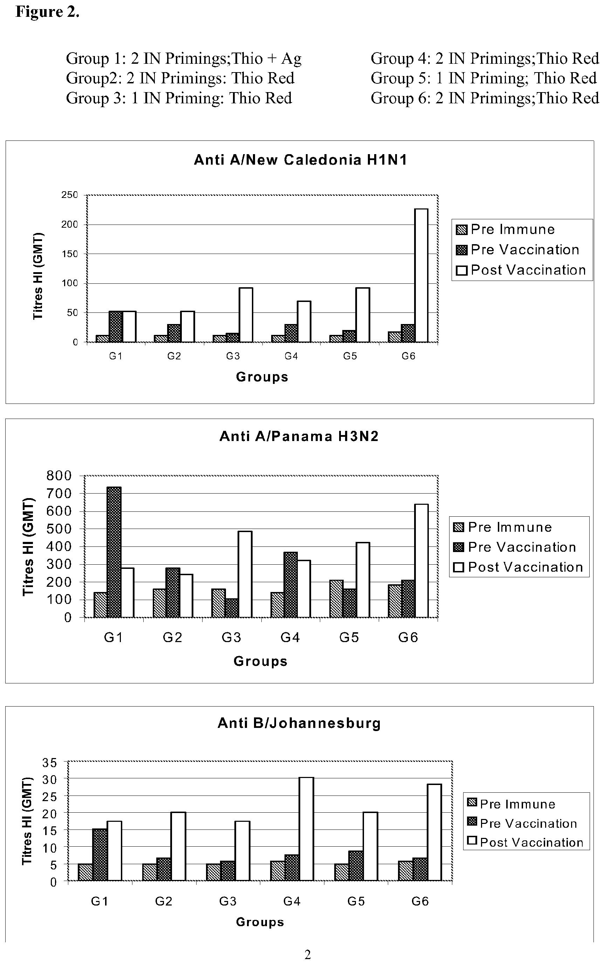

The HI results obtained in this experiment are shown in Figure 2 (Anti-Influenza Hemagglutination Inhibition Titers Induced in Pigs Primed with a Variety of Antigen Doses and Vaccinated with 3 Micrograms Trivalent Influenza Antigen Plus or Minus Thiomersal by the Intradermal Route).

Relatively low HI titers are induced to the B strain in this experiment and the background against the A/H3N2 strain is high. A beneficial effect in terms of response to vaccination is observed when the priming dose is reduced. In almost all cases, reduction in the antigen concentration or number of priming doses (from the two primings with 50 µg) resulted in a heightened response to vaccination. While the response of the animals in Groups 1 and 2, which were primed twice with 50 µg, to vaccination is not so evident, it appears that the preservative-free antigen (Group 2) functions at least as well as Fluarix™ (Group 1) under these conditions. A strong response to vaccination with preservative-free trivalent influenza antigen administered by the ID route in the alternatively primed animals (Groups 3-6) is clear and this response is seen even in the B strain, although the HI titers remain low.

### Example 13 - Phase III double-blind, randomised, comparative study in children aged 6 months to <6 years

### 13.1 Methods

### 13.1.1 Study design

Children aged 6 months to <6 years, with or without an underlying chronic disease, and who had never previously been vaccinated against influenza, were included in the study.

Two doses of the study vaccine (TF influenza split vaccine prepared according to a method similar as that described in Example 5) or the control vaccine (thiomersal-reduced *Influsplit SSW*®/*Fluarix*™*,* containing <2.5 µg thiomersal per 0.5 ml dose) (0.25 ml for children 6 to 35 months old and 0.5 ml for children 36 months to <6 years old) were administered on Day 0 and Day 28 ± 2. Both vaccines contained 15 µg HA of each viral strain A/New Caledonia/20/99 (IVR-116) [H1N1], A/Panama/2007/99 (RESVIR-17) [H3N2] and B/Shangdong/7/97, i.e. the recommended strains for the Northern Hemisphere during the 2003/2004 influenza season.

### 13.1.2 Immunogenicity

Serum samples were to be collected prior to immunisation (Day 0), 21 days after the second vaccination (Day 49 ± 2), 3 months after the second vaccination (Day 118/Month 4) and 6 months after the second vaccination (Day 208/Month 7). Sera were analysed by HA inhibition (HI) test, according to standard procedures. Antigens used for testing were the same as those included in the vaccine formulation (A/New Caledonia/20/99 (IVR-116) [H1N1], À/Panama/2007/99 (RESVIR-17) [H3N2] and B/Shangdong/7/97). Each serum was tested at a starting dilution of 1:10. Geometric mean titres (GMTs), SPRs (i.e. the percentage of subjects with a serum HI titre ≥ 1:40 after vaccination), SCRs (i.e. the percentage of subjects with either a pre-vaccination HI titre < 1:10 and a post-vaccination titre ≥ 1:40 or a pre-vaccination titre ≥ 1:10 and at least a fourfold increase in post-vaccination titre), and SCFs (i.e. the fold increase in HI GMTs post-vaccination compared to Day 0) were calculated. As guidelines for the immunogenicity assessment of influenza vaccines in children have not yet been established, serological results were evaluated according to the CHMP criteria for the assessment of influenza vaccines in adults aged 18-60 years: SPR >70%, SCR >40% and SCF >2.5 were considered as cut-off levels of vaccine immunogenicity. In addition, the seroprotection power (SPP; i.e. the proportion of subjects not seroprotected before vaccination [titre <1:40 on Day 0] who displayed a seroprotective titre of ≥ 1:40 after the second vaccination) was calculated as a further derived parameter.

### 13.2 Results

### 13.2.1 Reactogenicity and safety

Both vaccines presented a favourable safety profile.

### 13.2.2 Immunogenicity

Immunogenicity results for each age and vaccine groups are summarised in Table 9 and Table 10. Pre-vaccination GMTs for all three vaccine strains were within the same range in the two vaccine groups. Twenty-one days after the second vaccination, in the TF group, GMTs ranged between 71.3 and 283.0 for children aged 6-35 months and between 180.3 and 712.7 for those aged 36 months to <6 years. In the control group, GMTs ranged from 31.3 to 111.2 for children aged 6-35 months and from 165.1 to 529.8 for those aged 36 months to <6 years. After two doses, the CHMP criteria for the immunogenicity assessment of influenza vaccines in adults were met for both children aged 6 to 35 months and those aged 36 months to <6 years in the TF group (Table 9 and Table 10). In the control group, only the SCR and SCF criteria were met for both age groups. The SPR was higher than 70% for all three strains in older children (aged 36 months to <6 years) but did not exceed 65.9% for any of the three strains in younger children (aged 6 to 35 months).

In the longer follow-up period (i.e. at Month 4 and Month 7 post-vaccination), the immune response persisted, although at a lower level. Persistence was comparable for both vaccine and age groups.

**Table 9 - Summary of immunogenicity results pre-vaccination and 21 days after second vaccination (Total Vaccinated Cohort- Subjects aged 6-35 months)**

| | **TF (n=42)** | | **Control (n=41)** | |
|---|---|---|---|---|
| | **D0** | **PII (D49)** | **D0** | **PII (D49)** |
| **GMT (value, 95% CI)** | | | | |
| A/New Caledonia | 5.2 (4.8-5.5) | 71.3 (49.1-103.5) | 5.2 (4.8-5.5) | 31.3 (21.0-46.8) |
| A/Panama | 25.2 (13.4-47.4) | 283.0 (157.0-510.1) | 17.5 (9.6-31.6) | 111.2 (57.5-215.3) |
| B/Shangdong | 5.8 (4.9-6.9) | 113.2 (77.3-165.9) | 5.7 (4.7-7.0) | 45.8 (29.2-71.8) |
| **SPR (%, 95% CI)** | | | | |
| A/New Caledonia | 0.0 (0.0-8.4) | 73.8 (58.0-86.1) | 0.0 (0.0-8.6) | 53.7 (37.4-69.3) |
| A/Panama | 40.5 (25.6-56.7) | 97.6 (87.4-99.9) | 31.7 (18.1-48.1) | 65.9 (49.4-79.9) |
| B/Shangdong | 2.4 (0.1-12.6) | 85.7 (71.5-94.6) | 4.9 (0.6-16.5) | 65.9 (49.4-79.9) |
| **SPP (%, 95% CI)** | | | | |
| A/New Caledonia | - | 73.8 (58.0-86.1) | - | 53.7 (37.4-69.3) |
| A/Panama | - | 96.0 (79.6-99.9) | - | 50.0 (30.6-69.4) |
| B/Shangdong | - | 85.4 (70.8-94.4) | - | 64.1 (47.2-78.8) |
| **SCR (%, 95% CI)** | | | | |
| A/New Caledonia | - | 73.8 (58.0-86.1) | - | 51.2 (35.1-67.1) |
| A/Panama | - | 95.2 (83.8-99.4) | - | 63.4 (46.9-77.9) |
| B/Shangdong | - | 83.3 (68.6-93.0) | - | 65.9 (49.4-79.9) |
| **SCF (value, 95% CI)** | | | | |
| A/New Caledonia | - | 13.8 (9.6-19.8) | - | 6.1 (4.1-9.0) |
| A/Panama | - | 11.2 (8.3-15.2) | - | 6.4 (4.5-9.0) |
| B/Shangdong | - | 19.5 (13.5-28.2) | - | 8.0 (5.4-11.9) |

The Total Vaccinated Cohort included 157 subjects, but as immunological results were not available for 12 subjects, the actual size of this cohort for the immunological analyses was 145.
TF= thiomersal free vaccine (study vaccine)
Control=*Influsplit SSW*®/*Fluarix*™
D= day, PII= post-vaccination 2, GMT= geometric mean titre; CI= confidence
interval; SPR= seroprotection rate, SPP= seroprotection power, SCR= seroconversion rate, SCF= seroconversion factor

**Table 10 - Immunogenicity results pre-vaccination and 21 days after second vaccination (Total Vaccinated Cohort- Subjects aged 36 months- <6 years)**

| | **TF (n=29)** | | **Control (n=33)** | |
|---|---|---|---|---|
| | **D0** | **PII (D49)** | **D0** | **PII (D49)** |
| **GMT (value, 95% CI)** | | | | |
| A/New Caledonia | 25.4 (13.0-49.7) | 561.0 (230.7-1363.7) | 10.0 (6.1-16.3) | 191.3 (101.6-360.2) |
| A/Panama | 78.1 (37.1-164.5) | 712.7 (432.4-1174.6) | 56.0 (28.3-110.6) | 529.8 (303.7-924.2) |
| B/Shangdong | 6.0 (4.8-7.4) | 180.3 (113.8-285.5) | 7.8 (5.7-10.5) | 165.1 (93.9-290.3) |
| **SPR (%, 95% CI)** | | | | |
| A/New Caledonia | 48.3 (29.5-67.5) | 82.8 (64.2-94.2) | 21.2 (9.0-38.9) | 84.8 (68.1-94.9) |
| A/Panama | 69.0 (49.2-84.7) | 96.6 (82.2-99.9) | 63.6 (45.1-79.6) | 97.0 (84.2-99.9) |
| B/Shangdong | 3.4 (0.1-17.8) | 86.2 (68.3-96.1) | 12.1 (3.4-28.2) | 90.9 (75.7-98.1) |
| **SPP (%, 95% CI)** | | | | |
| A/New Caledonia | - | 66.7 (38.4-88.2) | - | 80.8 (60.6-93.4) |
| A/Panama | - | 88.9 (51.8-99.7) | - | 91.7 (61.5-99.8) |
| B/Shangdong | - | 85.7 (67.3-96.0) | - | 89.7 (72.6-97.8) |
| **SCR (%,** | | | | |
| **95% CI)** | | | | |
| A/New Caledonia | - | 75.9 (56.5-89.7) | - | 84.8 (68.1-94.9) |
| A/Panama | - | 79.3 (60.3-92.0) | - | 84.8 (68.1-94.9) |
| B/Shangdong | - | 86.2 (68.3-96.1) | - | 87.9 (71.8-96.6) |
| **SCF (value, 95% CI)** | | | | |
| A/New Caledonia | - | 22.1 (12.2-40.0) | - | 19.1 (12.5-29.4) |
| A/Panama | - | 9.1 (5.5-15.1) | - | 9.5 (6.3-14.2) |
| B/Shangdong | - | 30.2 (20.4-44.5) | - | 21.3 (14.3-31.6) |

The Total Vaccinated Cohort included 157 subjects, but as immunological results were not available for 12 subjects, the actual size of this cohort for the immunological analyses was 145.
TF= thiomersal free vaccine (study vaccine)
Control= *Influsplit SSW*®/*Fluarix*™
D=day, PII= post-vaccination 2, GMT= geometric mean titre; SPR= seroprotection rate, SPP= seroprotection power, SCR= seroconversion rate, SCF= seroconversion factor

### 13.3 Conclusion

Both the new thiomersal-free formulation of Fluarix™ and the thiomersal-reduced Fluarix™ (control) were immunogenic and presented a good safety profile.

The TF vaccine was shown to fulfil all three CHMP criteria defined for adults both in children aged 6 to 35 months and in children aged 36 months to <6 years and for all 3 strains. Immunogenicity was shown to be higher in children younger than 3 years of age receiving 2 doses of the TF vaccine compared with those receiving 2 doses of the control vaccine.

## Claims

1. A mercurial preservative-free immunogenic composition for use in prophylaxis of influenza infection or disease in a child of between 6 months and 36 months of age, wherein said composition comprises an aqueous inactivated influenza virus preparation comprising haemagglutinin (HA) and α-tocopherol succinate in an amount sufficient to stabilize said HA, wherein the preparation is a split or sub-unit virus antigen preparation and is a trivalent influenza virus preparation comprising 2 A strains and 1 B strain HA or a tetravalent influenza virus preparation comprising 2 A strains and 2 B strains HA and wherein the composition does not comprise an adjuvant.

2. The mercurial preservative-free immunogenic composition for use according to claim 1, wherein the α-tocopherol succinate is present in an amount such that the HA of said preparation remains stable for at least 6 months after said preparation is produced as determined by the presence of an amount of HA detectable by SRD assay.

3. The mercurial preservative-free immunogenic composition for use according to claim 1, wherein the concentration of HA antigen for each strain of influenza is 1-100 µg per ml, as measured by a SRD assay.

4. The mercurial preservative-free immunogenic composition for use according to claim 1, wherein the concentration of HA antigen for each strain of influenza is about 15 µg per ml, as measured by a SRD assay.

5. The mercurial preservative-free immunogenic composition for use according to claim 1, wherein the concentration of HA antigen for each strain of influenza is less than 15 µg per ml, as measured by a SRD assay.

6. The mercurial preservative-free immunogenic composition for use according to claim 5, wherein the amount of HA antigen for each strain of influenza is between 6-9 µg per dose, as measured by a SRD assay.

7. The mercurial preservative-free immunogenic composition for use according to claim 1, wherein the immunogenic composition has a dose volume of about 0.5 ml or of about 0.25 ml.

8. The mercurial preservative-free immunogenic composition for use according to claim 1, wherein the immunogenic composition has a dose volume of between 0.2 to 0.45 ml.

## Patentansprüche

1. Immunogene Zusammensetzung frei von quecksilberhaltigem Konservierungsmittel zur Verwendung bei der Prophylaxe von Influenza-Infektion oder -Erkrankung in einem Kind im Alter von zwischen 6 Monaten und 36 Monaten, wobei die Zusammensetzung eine wässrige Zubereitung von inaktiviertem Influenzavirus umfasst, welche Hämagglutinin (HA) und α-Tocopherolsuccinat in einer Menge umfasst, die ausreichend ist, um das HA zu stabilisieren, wobei die Zubereitung eine Spalt- oder Untereinheit-Virusantigen-Zubereitung ist und eine trivalente Influenzavirus-Zubereitung, umfassend 2 A-Stämme und 1 B-Stamm HA, oder eine tetravalente Influenzavirus-Zubereitung, umfassend 2 A-Stämme und 2 B-Stämme HA, ist, und wobei die Zusammensetzung kein Adjuvans umfasst.

2. Immunogene Zusammensetzung frei von quecksilberhaltigem Konservierungsmittel zur Verwendung gemäß Anspruch 1, wobei das α-Tocopherolsuccinat in einer solchen Menge vorliegt, dass das HA der Zubereitung für wenigstens 6 Monate, nachdem die Zubereitung produziert wurde, stabil bleibt, bestimmt durch das Vorliegen einer Menge an HA, die mittels SRD-Assay detektierbar ist.

3. Immunogene Zusammensetzung frei von quecksilberhaltigem Konservierungsmittel zur Verwendung gemäß Anspruch 1, wobei die Konzentration von HA-Antigen für jeden Influenza-Stamm 1-100 µg pro ml beträgt, gemessen durch einen SRD-Assay.

4. Immunogene Zusammensetzung frei von quecksilberhaltigem Konservierungsmittel zur Verwendung gemäß Anspruch 1, wobei die Konzentration von HA-Antigen für jeden Influenza-Stamm etwa 15 µg pro ml beträgt, gemessen durch einen SRD-Assay.

5. Immunogene Zusammensetzung frei von quecksilberhaltigem Konservierungsmittel zur Verwendung gemäß Anspruch 1, wobei die Konzentration von HA-Antigen für jeden Influenza-Stamm weniger als 15 µg pro ml beträgt, gemessen durch einen SRD-Assay.

6. Immunogene Zusammensetzung frei von quecksilberhaltigem Konservierungsmittel zur Verwendung gemäß Anspruch 5, wobei die Menge an HA-Antigen für jeden Influenza-Stamm zwischen 6-9 µg pro Dosis beträgt, gemessen durch einen SRD-Assay.

7. Immunogene Zusammensetzung frei von quecksilberhaltigem Konservierungsmittel zur Verwendung gemäß Anspruch 1, wobei die immunogene Zusammensetzung ein Dosisvolumen von etwa 0,5 ml oder von etwa 0,25 ml hat.

8. Immunogene Zusammensetzung frei von quecksilberhaltigem Konservierungsmittel zur Verwendung gemäß Anspruch 1, wobei die immunogene Zusammensetzung ein Dosisvolumen von zwischen 0,2 bis 0,45 ml hat.

## Revendications

1. Composition immunogène dépourvue de conservateur mercuriel pour son utilisation dans la prophylaxie d'une infection ou maladie grippale chez un enfant âgé de 6 à 36 mois, dans laquelle ladite composition comprend une préparation aqueuse à base de virus de la grippe inactivé comprenant de l'hémagglutinine (HA) et du succinate d'α-tocophérol en une quantité suffisante pour stabiliser ladite HA, dans laquelle la préparation est une préparation antigénique à base de virus fragmenté ou sous-unitaire et est une préparation trivalente à base du virus de la grippe comprenant l'HA de 2 souches A et de 1 souche B ou une préparation tétravalente à base de virus de la grippe comprenant l'HA de 2 souches A et de 2 souches B et dans laquelle la composition ne comprend pas d'adjuvant.

2. Composition immunogène dépourvue de conservateur mercuriel pour son utilisation selon la revendication 1, dans laquelle le succinate d'α-tocophérol est présent en une quantité telle que l'HA de ladite préparation reste stable pendant au moins 6 mois après la production de ladite préparation, tel que déterminé par la présence d'une quantité de HA détectable par un dosage par SRD.

3. Composition immunogène dépourvue de conservateur mercuriel pour son utilisation selon la revendication 1, dans laquelle la concentration en antigène HA pour chaque souche du virus de la grippe est de 1 à 100 µg par mL, telle que mesurée par un dosage par SRD.

4. Composition immunogène dépourvue de conservateur mercuriel pour son utilisation selon la revendication 1, dans laquelle la concentration en antigène HA pour chaque souche du virus de la grippe est d'environ 15 µg par mL, telle que mesurée par un dosage par SRD.

5. Composition immunogène dépourvue de conservateur mercuriel pour son utilisation selon la revendication 1, dans laquelle la concentration en antigène HA pour chaque souche du virus de la grippe est inférieure à 15 µg par mL, telle que mesurée par un dosage par SRD.

6. Composition immunogène dépourvue de conservateur mercuriel pour son utilisation selon la revendication 5, dans laquelle la quantité d'antigène HA pour chaque souche du virus de la grippe est comprise entre 6 et 9 µg par dose, telle que mesurée par un dosage par SRD.

7. Composition immunogène dépourvue de conservateur mercuriel pour son utilisation selon la revendication 1, dans laquelle la composition immunogène a un volume de dose d'environ 0,5 mL ou d'environ 0,25 mL.

8. Composition immunogène dépourvue de conservateur mercuriel pour son utilisation selon la revendication 1, dans laquelle la composition immunogène a un volume de dose compris entre 0,2 et 0,45 mL.
